Europäisches Patentamt

European Patent Office

Office européen des brevets

(11) Publication number: **0 218 182 B1**

## EUROPEAN PATENT SPECIFICATION

(45) Date of publication of patent specification: **15.04.92**   (51) Int. Cl.⁵: **A61M  21/00**, A63H 3/00

(21) Application number: **86113500.2**

(22) Date of filing: **01.10.86**

(54) **A child calming toy with rhythmic stimulation.**

(30) Priority: **07.10.85 KR 12993**
          **27.01.86 US 822530**

(43) Date of publication of application:
**15.04.87 Bulletin  87/16**

(45) Publication of the grant of the patent:
**15.04.92 Bulletin  92/16**

(84) Designated Contracting States:
**AT BE CH DE ES FR GB GR IT LI NL SE**

(56) References cited:
**FR-A- 1 557 709**
**FR-A- 2 094 929**
**FR-A- 2 441 400**
**US-A- 4 136 685**
**US-A- 4 257 408**

(73) Proprietor: **Lee, Min Joo**
**Chosun International, Inc. Jangan-Dong**
**Dongdaemun-Ku Seoul(KR)**

(72) Inventor: **Lee, Min Joo**
**Chosun International, Inc. Jangan-Dong**
**Dongdaemun-Ku Seoul(KR)**

(74) Representative: **Glawe, Delfs, Moll & Partner**
**Patentanwälte**
**Postfach 26 01 62 Liebherrstrasse 20**
**W-8000 München 26(DE)**

## Description

The invention relates to pacification devices for children. a child pacifying device as recited in the introductory clause of claim 1.

Over the years considerable technology has developed around the solution to the problem of pacifying babies and small children. Generally, infantile unrest and later, the hyper-activity of small children, are naturally occurring phenomena which, to greater and lesser extents, appear to be "programmed into" human beings from birth. In particular, the response of infants to the outside world appears to be calculated to stimulate the sort of parental attention which would certainly have been required in the relatively dangerous environment in which man evolved.

Thus the classic pattern appears. An abandoned child cries, signalling his mother to devote some attention to him either in the form of hugging, cuddling, nursing or the like. Upon the application of one of these stimuli, the crying usually stops.

Above and beyond this it has been found in research with the related species that withholding such stimuli can have permanent adverse effects on personality development and the mental stability of the adult animal. Controlled studies have shown, for example, that primates brought up in sterile laboratory surroundings without any objects around them have been found to be significantly disadvantaged as compared to other animals of the same species which were provided with a form which they could hug and which included structure which they could protect themselves in.

While such work is relatively recent, most successful infant pacifiers have always simulated otherwise naturally occurring human interactions. A few examples of such devices include milk bottles, nipple pacifiers, and soft dolls. In the case of somewhat older children one may also add pets and a different class of devices calculated to stimulate and interest the mind of the child. Such devices include crib chimes, animated dolls, talking dolls and the like.

While all of the above devices do perform the desired function of pacifying children, they all suffer from various inadequacies. For example, nipple pacifiers, while they are quite inexpensive and may initially be effective, do essentially frustrate the natural expectations of the infant and, after a short time, are recognized and rejected. Stuffed toy animals, while soft and appealing in texture, are essentially passive and thus, particularly in the case of infants, are not very effective as the infant is unable to fully comprehend the device. To a limited extent the device can be improved by incorporating an audio device (such as a tape recorder) in a toy doll to add an audio stimulus to such a pacifier.

However, the stimulus is extremely complicated and not of a type likely to be understood or learned by a small infant. In addition, in the case of smaller children, talking dolls are likely to have the opposite of the desired effect, that is stimulating activity, instead of providing a calming influence. In addition, such talking dolls are likely to be expensive and complicated devices which are highly subject to breakdowns and sensitive to the abuse likely to be given to them by children.

It is known to provide a soft, huggable and pleasantly textured object, such as a teddy bear, with an audio cue of a simple periodic nature which is capable of being understood and anticipated by even very small infants and, in the case of small children, is identifiable with life functions of a real parent, companion or pet.

Devices of this kind known from prior art include an electromechanical device which generates a sound to simulate the heartbeat, but does not allow the child to feel the heartbeat at the outer surface of the stuffed doll body. In FR-A-2 441 400, which represents the pertinent state of the art, there is disclosed a heartbeat mimicking device comprising a hammer striking two membranes to produce a double hollow sound imitating the beating of a heart. This entails the adverse effects that in contrast to a real heartbeat there is audible sound and an impact-originated abrupt sensation in the mechanism. Another disadvantage is the fact that the apparatus has to be switched on which normally would need help by an adult.

The invention is based on the problem to create a child pacifying device in the form of a stuffed doll or animal in order to give to the child a feeling of a heartbeat as realistic as possible.

The invention solves this problem by the characterizing features of the main claim.

One aspect of the invention involves the use of an electromagnet which attracts a relatively heavy bob weight on a leaf spring. Because the leaf spring is secured to a casing inside the stuffed animal, the casing tends to "beat" like a heart. This results in an improvement in that the above-mentioned disadvantages of audible sound and impact-originated abrupt sensation are eliminated.

Still another feature of the invention is the fact that the heart is stimulated to beat in response to hugging of the animal due to the presence of a push-button switch which extends from the front of the heartbeat mechanism. The heartbeat mechanism is positioned for the switch to be pushed in when the animal is hugged, thus making the heartbeat mechanism beat in response to the proximity of a child or other person hugging the animal.

This proximity actuation of the heartbeat mechanism is important for a realistic heartbeat simulation. Because of limited power resources of the battery it is not feasible to let the mechanism run all the time. On the other hand, a manual switch for actuating the mechanism to be operated by the child would impair the realism of the heart-beat simulation. The necessity to operate this switch would remind the child of the artificiality of the heartbeat. Young children even would not be capable of actuating the switch. The proximity actuation creates the illusion of a permanent beating heart and is therefore an important feature for solving the inventional task.

## BRIEF DESCRIPTION OF DRAWINGS

One way of carrying out the invention is described in detail below with reference to drawings which illustrate only specific embodiments of the invention, in which:

Figure 1 is a perspective illustrating the position of a heart-beat simulator contained within the inventive toy;

Figure 2 is a perspective view of the simulator;

Figure 3 is a cross sectional view along lines 3-3 of Figure 2;

Figure 4 is a view of the vibrator circuit of the simulator;

Figure 5 is a is a plan view of the heart beat transducer;

Figure 6 is a plan view along lines 6-6 of Figure 5;

Figure 7 is a graph of the excitation voltage to the transducer; and

Figure 8 is a schematic of the vibrator circuit that outputs the waveform of Figure 7 to the transducer.

## BEST MODE FOR CARRYING OUT THE INVENTION

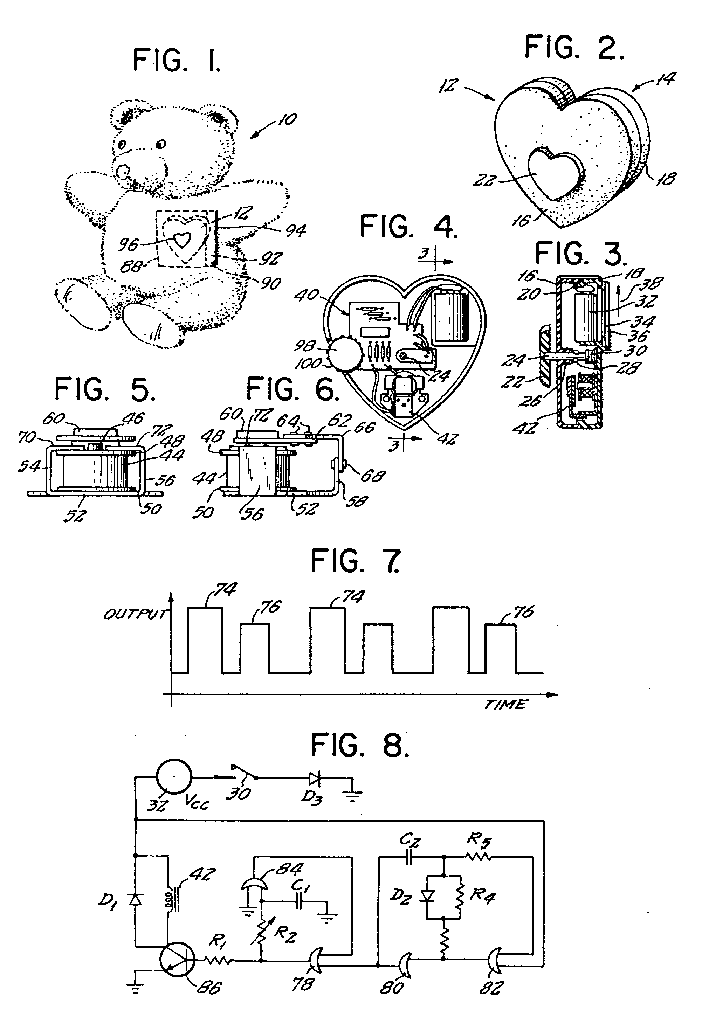

Referring first to Figures 1-2, the preferred embodiment of the invention is directed to dolls and toy animals including a subsystem which provides the impression of actual heart beats. It is believed that a doll, such as toy animal or teddy bear 10, with a heart-beat simulator 12 will induce in the person playing with it feelings of contentment, warmth and comfort, and aesthetic satisfaction similar to those he would experience if he were handling or being played with by a living person. Housing 14 comprises a front cover 16 and a rear cover 18 which may engage each other via an overhanging edge 20 on the rear cover as illustrated in figure 3. As can be seen most clearly in Figures 2 and 3 a heart shaped push button actuator 22 is slidingly mounted in the front cover 16 for movement toward and away from rear cover 18. This movement is achieved by providing a support pin 24 which is secured to actuator 22 and slides in a sleeve 26 which is secured to and integral with front cover 16. Actuator 22 together with support pin 24 is prevented from falling out from housing 14 by a stop member 28 which is integral with pin 24.

As can be seen most clearly in figure 3, support pin 24 directly overlies a switch 30 which is wired to actuate an electronic pulsing circuit powered by a battery 32. Replacement of worn batteries is accommodated by a slide battery cover 34 of conventional design and which incorporates gripping surface 36. Cover 34 is configured to be slidingly removed in the direction indicated by arrow 38 upon the application of appropriate pressure to gripping surface 36. Finally, the housing contains an electronic pulsating circuit 40 and a heart beat simulating transducer 42.

Transducer 42, as shown in Figure 5-6, comprises an electromagnet 44 which includes a core 46 around which the windings of electromagnet 44 are wound. The windings are confined by a pair of spool ends 48 and 50 which serve as a means for securing electromagnet 44 to a support 52. Support 52 is made from a generally T-shaped sheet of ferromagnetic material such as steel. The sheet is bent to form a pair of magnetic flux conducting members 54 and 56 and a spring support 58 (figure 6).

Electromagnet 44 actuates a ferromagnetic bob 60 which is mounted on a ferromagnetic strip 62 by epoxy or any other suitable adhesive. Strip 62, in turn, is riveted by rivets 64 to spring 66 which, in turn, is riveted by rivets 68 to support 58. The spring constant of spring 66 is such that upon the application of a pulse to electromagnet 44, bob 60 will be attracted toward but will not make clicking contact with the top surfaces 70 and 72 of members 54 and 56.

A typical driving voltage is illustrated in figure 7. As can be seen from figure 7, the pulses alternate between a higher amplitude pulse 74 having a relatively low frequency content and a smaller amplitude pulse 76 having a relatively high frequency content, as illustrated in figure 7. As these pulses are applied to transducer 42, which in turn deflects the mass of bob 60 and results in causing relative movement of housing 14 with respect to bob 60, the impression of a beating heart is given by heart shaped housing 14 which vibrates backwards and forwards in the chest of bear 10.

Although many electronic circuits are capable of producing a suitable pulse train such as that illustrated in figure 7 or other pulse trains which will also provide a heart beat simulation, by way of illustration one such circuit will be described in conjunction with figure 8. In particular, the device comprises a number of gates 78, 80, 82 and 84. These gates together with a transistor 86 form a circuit for driving transducer 42. Actuation of the electronic circuit is achieved by closing of switch 30 which is illustrated in figures 3, and 4 and, in schematic form, in figure 8. The electrical values or catalog numbers of the various electrical elements described above are:

| | |
|---|---|
| $V_{cc}$ | 9 Volt Battery |
| $D_1, D_2, D_3$ | 1N 4148 diodes |
| $R_1$ | 2.2 Kilohm resistor |
| $R_2$ | 300 Kilohm variable resistor |
| $R_3$ | 1.5 Megohm resistor |
| $R_4$ | 4.7 Megohm resistor |
| $R_5$ | 1 Megohm resistor |
| $C_1$ | .0068 microfarad capacitor |
| $C_2$ | .0047 microfarad capacitor |

When it is desired to use the inventive child pacifying device, the simulator 12 is provided with a battery 32 by removal of slide battery cover 34 which, after insertion of the battery is put back on the housing. The simulator 12 is then placed in a pocket 88 which is sewn into bear 10. Pocket 88 is closed by mating hooks-and-piles-type strips 90 and 92 which are contained within the pocket just inside its opening 94. After the pocket is closed by bringing hooks-and-piles-type strips 90 and 92 into contact with each other, the teddy bear is then ready to be enjoyed by the child. If desired an outer heart 96 may be sewn to the body of the teddy bear to cause a child to associate the heart beat with, for example, an applique heart 96. In the alternative applique heart 96 may be sewn to an item of clothing to be worn by the bear 10.

When the child either hugs the bear or feels its heart, it causes actuator 22 to drive pin 24 toward switch 30 closing the electrical circuit and commencing the generation of the pulses illustrated in figure 7 to the transducer illustrated in figures 5 and 6. As discussed above, this results in the perception of a pulsating heart within toy bear 10.

If desired, it is possible to regulate the strength of the heart beats in accordance with the power of the battery or, perhaps, the desire to minimize the strength of the heart beat to allow a particular child to sleep by regulation of the value of variable resistor $R_2$ to by rotation of a knurled knob 98 which includes serrations 100 to allow for easy rotation of the same. Naturally, whenever the child stops hugging bear 10 perhaps because it has fallen asleep or has stopped playing with the bear, the push button switch 30 automatically springs back to the unactuated position, thus conserving power in the battery.

While an illustrative embodiment of the invention has been described, it is, of course, understood that various modifications of the invention will be obvious to those of ordinary skill in the art. Such modifications are within the scope of the invention which is limited and defined only by the appended claims.

## Claims

1. A child pacifying device, comprising:
   a) a soft doll body (10);
   b) a container volume (88) defined in said body (10) and accessible through an opening (94) in the outer surface of said body (10);
   c) an electromagnetic pulsating device (12) disposed within said volume, having an electromagnet (44), support means (52, 58) secured to said electromagnet (44), a spring (66) secured in said support means (52, 58), and a bob weight (60) secured to said spring (66) and positioned to be attracted by said electromagnet (44); and
   d) switch means (30) for actuating said pulsating device (12),
   characterized in that the spring constant of the spring (66) is such that when the bob weight (60) is attracted by the electromagnet (44) will not make any clicking contact and that said switch means (30) is actuating said pulsating device (12) upon the application of pressure to the outer surface of said pulsating device (12).

2. A device as in claim 1, characterized in that said switch means (30) comprises a push button (22) which extends from the front (16) of said pulsating device (12).

3. A device as in claim 1 or 2, characterized in that said opening (94) may be closed.

4. A device as in claim 3, characterized in that said opening (94) may be closed by a pair of mating hooks-and-piles-type strips (90, 92) contained on opposite sides of said opening (94) and positioned not to be visible from the outside of said body (10) when said opening (94) is closed.

5. A device as in any of claims 1 to 4, characterized in that said spring (66) is secured to said housing (14) of said pulsating device (12).

6. A device as in claim 5, characterized in that said spring (66) is a leaf spring.

7. A device as in any of claims 1 to 6, characterized in that said electromagnet (44) is driven by an electronic circuit which is turned on and off by said push button switch (22, 30).

8. A device as in claim 7, characterized in that said electronic circuit outputs a series of pulses (74, 76).

9. A device as in claim 8, characterized in that said pulses (74, 76) alternate between a relatively strong pulse (74) and a relatively weak pulse (76).

10. A device as in any of claims 8 or 9, characterized in that said pulses (74, 76) alternate between pulses of relatively high frequency content and relatively low frequency content.

11. A device as in any of chaims 6 to 10, characterized in that said pulses (74, 76) comprise a series of two pulse groups, each of said pulse groups beginning with a relatively high amplitude and low frequency content pulse followed by a relatively low amplitude and high frequency content pulse.

## Revendications

1. Dispositif pour apaiser les enfants, comprenant
   a) un corps mou de poupée (10);
   b) un volume de logement (88) défini dans ledit corps (10) et accessible par une ouverture (94) dans la surface extérieure dudit corps (10);
   c) un dispositif pulsatoire électromagnétique (12) disposé dans ledit volume, comprenant un électro-aimant (44), des moyens de support (52, 58) fixés audit électro-aimant (44), un ressort (66) fixé dans lesdits moyens de support (52, 58) et un contrepoids d'équilibrage (60) fixé audit ressort (66) et disposé de façon à être attiré par ledit électro-aimant (44); et
   d) des moyens interrupteurs (30) pour l'actionnement dudit dispositif pulsatoire (12),
   caractérisé en ce que la constante de rappel du ressort (66) est telle que quand le contrepoids d'équilibrage (60) est attiré par l'électroaimant (44), il ne produise pas de contact générateur de bruit sec, et en ce que lesdits moyens interrupteurs (30) actionnent ledit dispositif pulsatoire (12) lorsqu'une pression est exercée sur la surface extérieure dudit dispositif pulsatoire (12).

2. Dispositif selon la revendication 1, caractérisé en ce que lesdits moyens interrupteurs (30) comprennent un bouton-poussoir (22) qui fait saillie sur le devant (16) dudit dispositif pulsatoire (12).

3. Dispositif selon la revendication 1 ou 2, caractérisé en ce que ladite ouverture (94) peut être fermée.

4. Dispositif selon la revendication 3, caractérisé en ce que ladite ouverture (94) peut être fermée par une paire de rubans appariés du type à crochets et à boucles (90, 92), contenus sur les côtés opposés de ladite ouverture (94) et placés de façon à ne pas être visibles de l'extérieur dudit corps (10) lorsque ladite ouverture (94) est fermée.

5. Dispositif selon l'une quelconque des revendications 1 à 4, caractérisé en ce que ledit ressort (66) est fixé audit boîtier (14) dudit dispositif pulsatoire (12).

6. Dispositif selon la revendication 5, caractérisé en ce que ledit ressort (66) est un ressort à lame.

7. Dispositif selon l'une quelconque des revendications 1 à 6, caractérisé en ce que ledit électro-aimant (44) est commandé par un circuit électronique qui est activé et désactivé par ledit interrupteur à bouton-poussoir (22, 30).

8. Dispositif selon la revendication 7, caractérisé en ce que ledit circuit électronique produit en sortie une série d'impulsions (74, 76).

9. Dispositif selon la revendication 8, caractérisé en ce que lesdites impulsions (74, 76) alternent entre une impulsion relativement forte (74) et une impulsion relativement faible (76).

10. Dispositif selon la revendication 8 ou 9, caractérisé en ce que lesdites impulsions (74, 76) alternent entre des impulsions de contenu de fréquence relativement élevé et de contenu de fréquence relativement bas.

11. Dispositif selon l'une quelconque des revendications 8 à 10, caractérisé en ce que lesdites impulsions (74, 76) comprennent une série de groupes de deux impulsions, chacun de ces groupes d'impulsions commençant par une impulsion d'amplitude relativement forte et de bas contenu de fréquence, suivie d'une impulsion d'amplitude relativement faible et de contenu de fréquence élevé.

## Patentansprüche

1. Beruhigend auf ein Kind einwirkende Vorrichtung, die aufweist:
   a) einen weichen Puppenkörper (10);
   b) ein in diesem Körper (10) abgegrenztes Behältervolumen (88), das durch eine Öffnung (94) in der äußeren Oberfläche des Körpers (10) zugänglich ist;

c) eine innerhalb dieses Volumens angeordnete elektromagnetische Pulsiereinrichtung (12) mit einem Elektromagneten (44), mit an diesem Elektromagneten (44) befestigten Trageinrichtungen (52, 58), mit einer an diesen Trageinrichtungen (52, 58) befestigten Feder (66) und mit einem an dieser Feder (66) befestigten Gewicht (60), das so angeordnet ist, daß es von dem Elektromagneten (44) angezogen werden kann; und

d) eine Schalteinrichtung (30) zur Betätigung der Pulsiereinrichtung (12),

dadurch gekennzeichnet, daß die Federkonstante der Feder (66) dergestalt ist, daß, wenn das Gewicht (60) von dem Elektromagneten (44) angezogen wird, es keine anschlagende Berührung macht, und daß auf die Ausübung von Druck auf die äußere Oberfläche der Pulsiereinrichtung (12) hin die Schalteinrichtung (30) die Pulsiereinrichtung (12) betätigt.

2. Vorrichtung nach Anspruch 1, dadurch gekennzeichnet, daß die Schalteinrichtung (30) einen Druckknopf (22) aufweist, der sich von der Vorderseite (16) der Pulsiereinrichtung (12) erstreckt.

3. Vorrichtung nach Anspruch 1 oder 2, dadurch gekennzeichnet, daß die Öffnung (94) geschlossen werden kann.

4. Vorrichtung nach Anspruch 3, dadurch gekennzeichnet, daß die Öffnung (94) mittels eines Paars zueinander passender Klettstreifen (90, 92) geschlossen werden kann, die an gegenüberliegenden Seiten der Öffnung (94) enthalten sind und so angeordnet sind, daß sie von der Außenseite des Körpers (10) nicht sichtbar sind, wenn die Öffnung (94) geschlossen ist.

5. Vorrichtung nach einem der Ansprüche 1 bis 4, dadurch gekennzeichnet, daß die Feder (66) an dem Gehäuse (14) der Pulsiereinrichtung (12) befestigt ist.

6. Vorrichtung nach Anspruch 5, dadurch gekennzeichnet, daß die Feder (66) eine Blattfeder ist.

7. Vorrichtung nach einem der Ansprüche 1 bis 6, dadurch gekennzeichnet, daß der Elektromagnet (44) von einem elektronischen Schaltkreis angesteuert wird, der durch den Druckknopfschalter (22, 30) an- und ausgeschaltet wird.

8. Vorrichtung nach Anspruch 7, dadurch gekennzeichnet, daß der elektronische Schaltkreis eine Folge von Pulsen (74, 76) abgibt.

9. Vorrichtung nach Anspruch 8, dadurch gekennzeichnet, daß die Pulse (74, 76) zwischen einem relativ starken Puls (74) und einem relativ schwachen Puls (76) abwechseln.

10. Vorrichtung nach einem der Ansprüche 8 oder 9, dadurch gekennzeichnet, daß die Pulse (74, 76) zwischen Pulsen von relativ hohem Frequenzgehalt und relativ niedrigem Frequenzgehalt abwechseln.

11. Vorrichtung nach einem der Ansprüche 8 bis 10, dadurch gekennzeichnet, daß die Pulse (74, 76) eine Folge von zwei Pulsgruppen aufweisen, wobei jede der beiden Pulsgruppen mit einem Puls von relativ hoher Amplitude und geringem Frequenzgehalt beginnt, worauf ein Puls relativ niedriger Amplitude und mit hohem Frequenzgehalt folgt.

FIG. I.

FIG. 2.

FIG. 4.

FIG. 3.

FIG. 5.

FIG. 6.

FIG. 7.

OUTPUT

TIME

FIG. 8.